# DEMANDE DE BREVET EUROPEEN

(11) **EP 0 566 804 A1**
(43) Date de publication de la demande: **27.10.1993**
(21) Numéro de dépôt: 92401197.6
(22) Date de dépôt: 24.04.1992
(51) Int. Cl.: A01K 1/015, A61L 9/01

(54) **Mélange de matériaux à propriétés multiples pour litières pour chats et petits animaux**

(71) Demandeur: SETRIC INTERNATIONAL S.A., F-31450 Montgiscard (FR)
(72) Inventeur: Chelle, René, 31190 Grepiac (FR)

(57) **Abrégé**

Il est proposé la réalisation de litières pour chats et petits animaux composées d'un mélange de matériaux de nature différente dont les fonctions complémentaires assurent l'élimination des liquides et des odeurs associées. L'un des matériaux possède un grand pouvoir absorbant de manière à ralentir l'évaporation et le départ de molécules malodorantes qui l'accompagnent. L'autre matériau présente un caractère caverneux et peu absorbant : sa grande surface spécifique complète l'action du premier par l'élimination d'une partie de l'eau résiduelle par évaporation. Ce dernier matériau est traité par un bactéricide qui se fixe sur lui mais peut être relargué dans le milieu liquide. De même, un parfum peut lui être ajouté : adsorbé seulement de façon superficielle, il est ainsi facilement volatilisé et peut donc être employé à faible dose.

Le produit proposé peut être la litière complète ainsi définie, ou encore le matériau caverneux et peu absorbant traité comme précédemment qui constitue alors un additif susceptible d'être ajouté à toute autre litière.

## Description

La présente invention concerne des litières pour chats et petits animaux faites d'un mélange de grains d'un matériau absorbant et d'un matériau peu dense, caverneux, et non absorbant, éliminant l'eau des urines par évaporation et absorption et empêchant le développement des mauvaises odeurs par adjonction de bactéricides.

L'émission quotidienne d'urines et de fèces par les animaux de compagnie a conduit à la mise au point de diverses litières qui, disposées dans des bacs, permettent de recueillir les déjections de ces animaux.

Pour des raisons de confort domestique et d'hygiène, ces litières sont formées de produits dont le but est la disparition des liquides et des mauvaises odeurs. Ces dernières résultant de la prolifération de microorganismes, il était important d'utiliser des bactéricides qui, en limitant ou annulant leur présence, permettaient en même temps de faire disparaître ces odeurs.

Après des premiers essais qui se bornaient à utiliser des matériaux naturels plus ou moins absorbants, comme la terre, le sable ou la sciure, l'attention s'est portée sur deux types de matériaux aux qualités différentes et parfois complémentaires.

Il s'agit d'abord de matériaux absorbants, comme la Sépiolite et l'Attapulgite, argiles de type fibreux, absorbants et de résistance mécanique élevée. D'autres matériaux aux propriétés analogues ont été également utilisés : matières plastiques poreuses, matières d'origine végétale ou industrielle, mais aucun n'a détrôné les argiles dont le pouvoir absorbant est meilleur et dont la nature minérale ne constitue pas une source carbonée propice à la multiplication des microorganismes.

Mais ces argiles, en retenant l'eau des urines, retiennent aussi les bactéries et les matières fermentescibles sur lesquelles celles-ci peuvent se multiplier. Il en résulte le dégagement d'odeurs nauséabondes de fermentation lorsque ces litières minérales sont utilisées seules. Pour pallier ces inconvénients, de nombreux additifs sont commercialisés : bactéricides ou parfums.

L'utilisation de ces additifs pose des problèmes car ils sont fortement retenus sur un matériau absorbant tel que la Sépiolite ou l'Attapulgite en raison même des propriétés que l'on recherche en lui. De grandes quantités d'additifs sont alors nécessaires.

Le deuxième type de matériaux concerne des produits caverneux de grande surface spécifique et non absorbants, comme la Pouzzolane. Avec ces matériaux, l'élimination de l'eau des résines se fait par des mécanismes d'évaporation naturelle rapide.

Nous avons déjà proposé, dans un brevet précédent, BF N° 8806246, d'utiliser ces matériaux pour la confection de litière, en les associant à un bactéricide et à un parfum.

La grande surface spécifique des matériaux peu denses et caverneux comme la Pouzzolane, facilite la répartition des liquides et leur évaporation. L'association avec un parfum et un bactéricide est facile : les odeurs initiales sont masquées par le parfum, le bactéricide interdit la formation ultérieure de mauvaises odeurs.

Un parallèle entre les deux types principaux de matériaux décrits ci-dessus permet de relever un certain nombre de différences et de similitudes qui mettent en valeur leur complémentarité :
- La Pouzzolane possède une résistance mécanique originelle très élevée. Un matériau absorbant tel que la Sépiolite ou l'Attapulgite, soumis à un traitement thermique approprié, acquiert des qualités mécaniques acceptables, même quand il a absorbé de l'eau.
- Du point de vue densité, la Sépiolite et la Pouzzolane présentent des avantages semblables, car elles sont, toutes les deux, des matériaux légers.
- Du point de vue capacité à absorber l'eau, la Sépiolite peut absorber approximativement son propre poids d'eau. La Pouzzolane ne retient l'eau que dans la proportion de 10% de son poids et cette eau n'est pas absorbée, mais simplement retenue par simple adsorption sous forme d'un film de faible épaisseur à la surface du grain.
- Du point de vue capacité à laisser s'évaporer l'eau, la Pouzzolane présente un avantage sur la Sépiolite. Ainsi, à volume égal en matériau, l'eau résiduelle est deux fois plus importante dans la Sépiolite que dans la Pouzzolane.

Les effets de l'adjonction de bactéricide peuvent également être comparés. L'urine est une solution aqueuse dont le principal constituant est l'urée. Sous l'action des uréases bactériennes, celle-ci se décompose facilement en ammoniac. Au bout de quelques heures après le début de son utilisation, la litière est bactériologiquement souillée par les premières déjections des animaux. Le dégagement malodorant de l'ammoniac, accentué par l'évaporation de l'urine, pose problème.

L'utilisation d'un bactéricide permet d'éviter ou de limiter la formation de ces produits volatiles. L'addition de bactéricides à des matériaux non caverneux et absorbants comme la Sépiolite ou l'Attapulgite suppose l'utilisation de quantités importantes de ces bactéricides du fait de la capacité d'absorption forte de ces matériaux. La Pouzzolane offre l'avantage, car peu absorbante, d'être efficacement antibactérienne avec un minimum de bactéricide, tout comme elle peut être efficacement parfumée avec une très faible quantité de molécules odorantes.

Pour profiter des propriétés complémentaires des deux types de matériaux, nous proposons d'utiliser des mélanges de matériaux peu absorbants (comme la Pouzzolane) et absorbants (comme la Sépiolite ou l'Attapulgite) pour la préparation de litières pour chats et petits animaux. Les noms de ces matériaux seront utilisés comme support à nos démonstrations pour leur caractère exemplaire. Mais notre invention concerne tous les matériaux utilisables dans les mêmes fonctions d'absorption ou d'évaporation des urines de chat.

C'est ainsi, par exemple, que l'évaporation accélérée de l'eau, favorisée par les produits peu absorbants, et qui s'accompagne du départ de molécules malodorantes, peut être suffisamment ralentie par l'association avec un matériau absorbant.

Le mécanisme d'action de ces mélanges peut être décrit de la manière suivante : l'eau libre, laissée par le matériau à grande surface spécifique comme la Pouzzolane, et qui ne se serait pas évaporée, est absorbée par le matériau absorbant, par exemple la Sépiolite. L'eau pénètre à l'intérieur de ce dernier où le développement bactérien devient plus difficile : tout développement bactérien superficiel étant par ailleurs exclu en raison du traitement aseptisant procuré par la Pouzzolane traitée ou tout autre matériau aux propriétés équivalentes et traité de la même façon. Celui-ci joue également le rôle de support de parfums. Les qualités suivantes sont dès lors combinées : désinfection, disparition des odeurs, maintien d'une litière sèche.

La préparation suivante est donnée à titre d'exemple non limitatif.

Pour fabriquer 150 kg de litière, on mélange 135 kg de Sépiolite brute à 15 kg de Pouzzolane traitée. Ce traitement consiste en l'adjonction de bactéricide et de parfum. Des essais ont été effectués avec différents bactéricides donnant des résultats concordants. Celui qui a été le plus souvent utilisé est le Glyoxal (préparation à 40%) utilisé à différentes concentrations, notamment à la concentration de 1%. Le traitement peut être fait de différentes façons et notamment en pulvérisations. De la même manière, le parfum est ajouté par pulvérisation.

On procède au mélange mécanique des deux constituants (Sépiolite brute plus Pouzzolane traitée) et on garnit les bacs à litière avec ce mélange.

Pour vérifier l'efficacité, deux séries d'essais ont été réalisées :
1° En reconstituant de façon synthétique les conditions d'utilisation. Ainsi, on réalise une culture bactérienne sur urée (le tout en présence du support minéral) et l'on dose l'ammoniac dégagé. Avec les mélanges décrits ci-dessus, la quantité d'urée transformée ne dépasse pas 2%.
2° Dans les conditions réelles d'utilisation, on garnit les bacs des animaux avec les litières ainsi préparées. Celles-ci sont utilisées de façon courante par les animaux. On ne constate aucun dégagement d'odeur avant, dans le cas le plus défavorable, onze jours d'utilisation.

Compte tenu de ce qui est donné dans ce descriptif, nous présentons pour invention l'utilisation pour litières (chats et petits animaux) d'un mélange d'un matériau absorbant (comme la Sépiolite ou l'Attapulgite), et d'un matériau peu dense, caverneux et non absorbant, par exemple la Pouzzolane, traitée avec un bactéricide, par exemple le Glyoxal, et éventuellement parfumée par l'adjonction de molécules odorantes.

## Revendications

1. Litière pour chats et petits animaux caractérisée par le fait qu'elle est composée d'un mélange de matériaux de nature différente, ayant des fonctions différentes et complémentaires quant à l'élimination des liquides et des odeurs associées.

2. Litière pour chats et petits animaux, selon Revendication 1, caractérisée par le fait que l'un des matériaux possède un grand pouvoir absorbant ce qui ralentit l'évaporation et le départ de molécules malodorantes accompagnant cette évaporation.

3. Litière pour chats et petits animaux, selon Revendications 1 et 2, caractérisée par le fait qu'un autre matériau du mélange présente un caractère caverneux et peu absorbant, associé à une grande surface spécifique, complétant l'action du premier par élimination d'une partie du liquide résiduel par évaporation.

4. Litière pour chats et petits animaux, selon Revendications 1, 2 et 3, caractérisée par le fait que le matériau à grande surface spécifique est traité par un bactéricide qui se fixe sur lui et peut être relargué au contact de l'urine, ce qui empêche la prolifération des microorganismes et par voie de conséquence la formation des odeurs.

5. Litière pour chats et petits animaux, selon Revendications 1, 2, 3 et 4, caractérisée par le fait que le matériau à grande surface spécifique est traité par un parfum, adsorbé de façon superficielle et qui peut, grâce à sa facile volatisation, être suffisamment efficace à très faible dose.

6. Litière pour chats et petits animaux, selon Revendications 1, 2, 3, 4 et 5, caractérisée par le fait que les matériaux utilisés ont des caractéristiques physiques (granulométrie, densité, dureté), équivalentes et qu'ils peuvent être facilement mélangés de façon à conserver les meilleures qualités mécaniques et à optimiser les propriétés.

7. Litière pour chats et petits animaux, selon Revendications 1, 2, 3, 4, 5 et 6, caractérisée par le fait que les proportions relatives des différents matériaux peuvent varier de 10 à 90%, de façon à ce que toute l'urine de chaque émission puisse bénéficier de l'action spécifique de chaque matériau.

8. Litière pour chats et petits animaux, selon Revendications 1, 2, 3, 4, 5, 6 et 7, caractérisée par le fait que le matériau de type absorbant puisse être tout matériau naturel ou artificiel ayant cette propriété, la Sépiolite ou l'Attapulgite étant deux exemples non limitatifs, et que le matériau à grande surface spécifique puisse être également naturel ou artificiel, (la Pouzzolane étant là encore un exemple non limitatif).

9. Litière pour chats et petits animaux, selon Revendications 1, 2, 3, 4, 5, 6, 7 et 8, caractérisée par le fait que le bactéricide est le Glyoxal ou tout autre composé de même activité.

10. Litière pour chats et petits animaux, selon Revendications 1, 2, 3, 4, 5, 6, 7, 8 et 9, caractérisée par le fait que le matériau à grande surface spécifique traité, (par exemple la Pouzzolane traitée), constitue un additif susceptible d'être ajouté à toute litière d'origine différente, de façon à constituer un produit complémentaire à ajouter qui permet à l'utilisateur de faire lui-même le mélange convenable.
